# EUROPEAN PATENT APPLICATION

(11) **EP 3 671 305 A1**
(43) Date of publication of application: **24.06.2020**
(21) Application number: 18213639.0
(22) Date of filing: 18.12.2018
(51) Int. Cl.: G02B 21/00, A61B 3/113, A61B 1/313, A61B 1/00, A61B 1/32

(54) **EXOSCOPE SYSTEM AND USE OF SUCH AN EXOSCOPE SYSTEM**

(71) Applicant: Eberhard Karls Universität Tübingen, 72074 Tübingen (DE)
(72) Inventor: Eivazi, Shahram, 71272 Renningen (DE)
(74) Representative: Patentanwaltskanzlei Cartagena

(57) **Abstract**

The invention concerns the field of medical operations and especially an exoscope system (10) with a camera arrangement (30) for capturing the image of an operating field (100), said exoscope system (10) comprising a support system (20) for positioning the camera arrangement (30) above the operating field (100) and a viewing device (40) for displaying an image region (110, 111, 112) of the operating field (100).

The camera arrangement (30) comprises at least two camera units (32) which can be directed to capture at least two different image regions (110, 111, 112) of the operating field (100). The exoscope system (10) further includes a controller (60). Said controller (60) is designed to select an image region (110, 111, 112) from said at least two image regions (110, 111, 112), to display its picture on the viewing device (40) and to switch between the at least two image regions (110, 111, 112) on the viewing device (40) as a function of the detected eye movement, viewing direction (2) and/or head movement. Alternatively oder additionally the controller (60) is designed to generate a common picture by stitching together the pictures (110', 111', 112') of the image regions (110, 111, 112), to display a partial segment of this common picture on the viewing device (40) and to change the partial segment of the common picture shown on a viewing device (40) as a function of the detected eye movement, viewing direction (2) and/or head movement.

## Description

### FIELD OF APPLICATION AND STATE OF THE ART

The invention concerns an exoscope system with a camera arrangement and a viewing device for use in medical operations.

Such exoscope systems provide digital image data of the operating field during operations. The camera arrangement of known exoscope systems as well as of exoscope systems according to the invention is provided for capturing the image of the operating field. The exoscope system has a support system for positioning the camera arrangement above the operating field. The viewing device is provided for displaying a picture of an image region of the operating field which is captured by the camera arrangement.

Exoscope systems known from the prior art often have the drawback that the surgeon needs a relevant part of his time for adjusting the camera arrangement in order to capture the specific region of current interest. Such adjustments usually take place several times during a surgical operation and interrupt the surgeon's handling of the surgical tools.

### OBJECT OF THE INVENTION AND SOLUTION

It is the object of this invention to provide an advanced exoscope system which decreases the need of adjusting and repositioning and which thus helps surgeons to concentrate on the operation and on handling the surgical tools.

An exoscope system according to the invention comprises in accordance with known exoscope systems a camera arrangement and a viewing device. The camera arrangement is mounted to a support system for positioning the camera arrangement above the operating field, usually in a distance between 20 cm and 70 cm. This camera arrangement digitally captures the image of an operating field from this position.

The viewing device is used for displaying a picture of the operating field which is captured by the camera arrangement. The viewing device is at least used by the surgeon, but can be designed for additional viewers also. The exoscope system according to the invention is primarily intended for the surgical fields of neurosurgical operations, ophthalmic operations and ENT operations as well as of plastic or reconstructive operations and dental operations.

A potential viewing device especially for usage by the surgeon only, could be a head mounted device with integrated displays like it is known from AR and VR systems. Another viewing device for usage by one person only is an ocular or preferably a pair of oculars each having an integrated display. As alternative or additional viewing device a regular display can be used like a flat display with a screen diagonal with preferably between 10" and 60". Such a regular display can be used by other participants in case the surgeon uses oculars or a head mounted device, but in another embodiment this regular screen can also be the only viewing device also being used by the surgeon.

The camera arrangement of the exoscope system according to the invention comprises at least two camera units which can be aligned for capturing at least two different image regions of the operating field. The camera units together capture a continuous large image area, whereby the individual image regions of the respective camera units can adjoin flush or overlap each other.

The exoscope system includes a controller for displaying a picture derived from the captured pictures by the multiple cameras. The controller also comprises a detection system for detecting eye movement, viewing direction and/or head movement of an operator, especially of the surgeon. The controller is enabled to process the picture data of the operating field from the multiple camera units in order to selectively show partial images according to one or both of the following modes of operation.

In a first possible mode the controller selects an image region from said at least two image regions and displays the regions' picture on the viewing device, wherein the controller is designed to switch between the at least two image regions on the viewing device as a function of the detected eye movement, viewing direction and/or head movement.

In a second possible mode the controller generates a common picture by stitching together the pictures of the image regions and displays a partial segment of this common picture on the viewing device. This partial image is a usually rectangular part of the stitched common picture and contains the region of the operating field currently relevant for the surgeon. The controller is designed to change the partial segment of the common picture shown on viewing device as a function of the detected eye movement, viewing direction and/or head movement. This change of the partial segment is usually a stepwise or scrolling movement on the stitched common picture such that the surgeon is able to move over the stitched common image by eye or head control.

The detection system for detecting eye movement, viewing direction and/or head movement can be designed in various ways. Advantageous the eye tracking system is part of a head mounted structure worn by the surgeon, for example part of a head mounted display (HMD). One preferred embodiment comprises one or more infrared lights per eye as well as one infrared camera pointing at the eye and capturing the image in order to detect the position of the pupil. Such a system can also be integrated in the oculars.

Alternatively or additionally the detection system can be capable to detect the orientation and/or position of the surgeon's head. This can be achieved by an external camera system that tracks the surgeon's movements. To enhance such tracking with an external camera an external infrared projector can be used for projecting an infrared pattern like a grid, which is then captured by an infrared camera.

As an alternative to the external tracking system, the detection of the orientation and/or position of the head of the surgeon can be achieved by using a head device worn by the surgeon and including sensors as for example acceleration sensors and magnetic compass sensors or cameras.

The data received from the camera arrangement and from the detection system is processed in the controller in one of the two modes mentioned above. In both modes the controller selectively shows partial picture data from the multiple camera units on the viewing device. Which picture data is being shown is selected on basis of commands from the surgeon derived from his head movement and/or eye movement. As multiple camera units are being used, the image data is of high quality. Furthermore the multiple camera units provide different camera positions and/or different angles in case they are set to converging viewing directions. Therefore the image quality especially of large operating fields is better than by using one single camera unit. Due to the large region covered by the multiple camera units, it is usually not necessary to reposition the camera arrangement during the operation.

In the first mode mentioned above the controller switches between the different pictures of the image regions so that usually the picture of only few camera units and especially of only one active camera unit is being shown on the viewing device while the pictures of the other camera units are hidden or not even captured. By moving his head or his eyes the surgeon can change the active camera unit and the picture on the viewing device is being exchanged accordingly.

In the second mode mentioned above the controller internally generates a common picture containing the picture data from the multiple camera units and especially of all camera units. These pictures are put together by using so called image stitching. As the position of the cameras is usually known to the controller, such an image stitching can be carried out in very good quality. The resulting common picture covers a large part of the full operating field in high resolution. Based on the eye movement or head movement a segment of this large common picture is shown on the viewing device. By turning his head or by moving his gaze the surgeon can move this segment to a new area of interest.

The camera units of the exoscope's camera arrangement can be each designed as stereo camera units with two individual cameras. Usually each individual camera of said two cameras has its own lens system and its own image sensor, which in particular is preferably a CCD image sensor or CMOS image sensor. Furthermore each individual camera of said two cameras can have a motor-driven focus unit.

If the camera units are provided as stereo camera units with two individual cameras, the viewing device or at least one viewing device in case of a system with multiple viewing devices, is designed as stereoscopic viewing device. In case of oculars and head mounted displays such two displays or a common display with different display regions for the two eyes of the surgeon can be used. In case of a regular screen the exoscope system can be provided with at least one shutter goggle alternately blocking the view of the eyes and changing the image on the screen between the two cameras of the currently active stereo camera unit accordingly, such that it generates a stereoscopic image on the screen. The switching between the left eye image and the right eye image usually takes place with at least 25 Hz.

Although an exoscope system according to the invention can have two camera units, especially two camera units each having two cameras for stereoscopic view, it is preferred that the camera arrangement of the exoscope has three or more camera units, each preferably comprising to two cameras for stereoscopic view.

In case of exact three camera units those camera units are preferably arranged chain-like one after another. With more than three camera units those camera units can be arranged in a two-dimensional matrix, for example nine camera units forming a 3 x 3 matrix or five camera arranged in a cross-shape.

As the digital camera sensors are quite cheap nowadays it is also possible to have larger numbers of cameras, for example 25 cameras in a 5 x 5 matrix pattern.

One main aspect of the invention is that it allows the surgeon to let the camera arrangement in fixed position at least most of the time during the surgical operation. The multiple camera units provide a large field of view even without being moved.

However it can be advantageous if the at least two camera units of the camera arrangement are movable relative to each other and can be fixed in a plurality of relative arrangements. This can be used for initially set up of the cameras prior to the surgical operation, for example depending on the distance of the camera arrangement relatively to the operation field. There are multiple options regarding the movability. In one embodiment the camera units can be tilted relatively to each other. Additionally or alternatively the camera units can be movable linearly to each other in order to change the distance between two adjacent camera units.

The fixing of the camera units can be fully variable regarding the relative position, especially by using a clamping mechanism or some kind of frictionally engaging fixing element. In another embodiment the camera units can be fixed relatively to each other in distinct stepped positions. In this case a positive fixing is possible.

It is recommended that the camera units can be moved relatively to each other directly manually by gripping a gripping portion assigned to a camera unit and apply a force relative to another camera unit. Preferably an electromotor is provided to assist the manual movement. Said electromotor can then also provide the mechanism for fixing the camera units relatively to each other.

Although only preferred for specific use cases a further embodiment is possible with an electromotor which is not only assisting manual movement of the camera units relatively to each other but to move the camera units independently from manually applied force on basis of software commands from the controller. Such a mode of operation can especially be useful in combination with a zoom functionality of the exoscope system. When zooming in, the size of the image regions decrease. In order to provide a large continuous image on basis of the pictures of all camera units even in zoomed state, a software controlled tilting of camera units adjacent to the active camera unit can be helpful.

As the operation field is usually smaller than the camera arrangement and the different image regions are spaced from each other less than adjacent camera units are spaced from each other, during usage the camera units preferably are arranged such that they have converging viewing directions. The angle between the viewing directions of adjacent camera units can be fixed or can be adjustable by above described manually, motoric assisted or by pure motoric movement.

It is preferred that the exoscope system according to the invention comprises at least one light source for illuminating of the operating field. In this case the controller is preferably designed to adjust the brightness of the at least one light source as a function of the detected eye movement, viewing direction and/or head movement. If the surgeon gazes dark parts of the picture on the viewing device the brightness is raised. If the surgeon gazes light parts of the picture on the viewing device the brightness is lowered. Furthermore it is preferred that the controller is also adapted to adjust the focus of the camera units as a function of the detected eye movement, viewing direction and/or head movement.

A further function which can be provided by the controller in reaction to the detected eye movement, viewing direction and/or head movement is the marking or highlighting of areas in the displayed image region. Said marking or highlighting therefore takes place in reaction to an according command of the surgeon, wherein usually the location the surgeon looks at in the moment the command is given is being marked or highlighted.

The marking or highlighting can be helpful for the surgeon himself in order to be able to change quicker between different locations of the operating field, especially if these different locations are part of different image regions captured by different camera units. The marking or highlighting can also be helpful for other people participating in the surgical operation by watching those marked or highlighted regions on a large screen in the operation theater.

The controller preferably is capable to record the captures pictures as video sequence. This can apply to the current segment or the active camera only, or for the captured images of all camera units together. This functionality serves documentation purposes, especially quality documentation, quality assurance and quality control, and can also be helpful in teaching and training of surgeons.

As mentioned before the changing of the image regions or of the partial segment on the viewing device is accomplished by the detection of the surgeon's eye movement, viewing direction and/or head movement. A very simple option for this is a controller which monitors the eye movement and which changes the displayed image region if the users viewing direction is directed to a margin of the currently displayed image region. In the first mode of operation the displayed image region can then be exchanged against another image region captured by the camera unit adjacent to the current camera unit in the direction of said margin. In the second mode of operation the partial segment can be moved in the direction of the margin the surgeon looks at.

Alternatively it is also possible to use a controller designed to display control elements on the viewing device. These control elements can be selected or activated by the user as a function of the detected eye movement or head movement. Especially the viewing direction can be monitored in order to recognize the user directing his view on one of the control elements. If the user's viewing direction remains there for a few seconds, the corresponding function can be selected or activated.

The functions assigned to the controls elements can be a switching functions for changing the displayed image region or partial segment. Additionally or alternatively other functions can be assigned to such control elements displayed on the viewing device, for example control elements for changing the focus, the zoom level and/or the lighting.

As described above a support system is being part of the exoscope system of the invention. Said support system allows positioning the camera arrangement above the operating field. In some embodiments the viewing device such like oculars or a large screen can also be integrated in the same support system.

In one possible embodiment the support system is a floor based support system. It therefore comprises a bottom base for placement on the floor next to the operating table. A support arm extends upwardly from the base and from the side over the operating field. At the end of said arm the camera arrangement is mounted. The bottom base can have a plurality of rollers in order to make the bottom base together with the rest of the support system mobile. Preferably at least some of the rollers can be blockable by means of a blocking device acting as a brake.

In order to position the camera arrangement in the right position above the operating field, the support arm can have at least one joint to allow adaptability of the positioning and/or alignment of the camera arrangement. Just as the described regarding the tilting of the camera units relatively to each other, this joint can be provided for pure manually handling, for handling assisted by an electromotor or for pure electromotoric movement on basis of commands from the controller.

Alternatively or additionally the support arm can have at least one telescopic section which is variable in length and therefore also allows adaptability of the positioning and/or orientation of the camera arrangement. Just as the support arm joints described above, the telescopic mechanism can be provided for pure manually handling, for handling assisted by an electromotor or for pure electromotoric movement on basis of commands from the controller.

As an alternative to the floor based system, the support system can also be provided for mounting on the ceiling. In this case the support system is provided with a ceiling base and a support arm extending downwardly from the ceiling base and extending over the operating field. At the end of this support arm the camera arrangement is mounted. As described for the floor based variant, such a ceiling based variant can also be provided with one or more joints or one or more telescopic section, either for pure or assisted manual movement or pure electromotoric movement.

As a further alternative, the support system can also be provided for mounting on a wall. In this case the support system is provided with a wall base and a support arm extending over the operating field. At the end of this support arm the camera arrangement is mounted. As described for the floor based and ceiling based variant, such a wall based variant can also be provided with one or more joints or one or more telescopic section, either for pure or assisted manual movement or pure electromotoric movement.

Although the control of the exoscope system according to the invention takes place at least partially by detecting the eye movement, the viewing direction and/or the head movement in order to change between the images of the different camera units, other methods of control can also be provided additionally. For example mouth controlled device or a foot control device, especially designed as a pedal, can be provided for further functionality. Moreover these additional input devices can be used in context of the switching between the image regions or the changing of the displayed segment specifically to enable the usually disabled functionality or specifically to disable the usually enabled functionality.

This avoids unintentional changes on the viewing device as for example such changes which only take place after the surgeon has actively enabled changes. Another approach can be threshold values regarding the eye movement or head movement to distinguish between viewing device control movements of the surgeon and his other movements. Furthermore it is possible to distinguish such movements on basis of machine learning algorithms.

### SHORT DESCRIPTION OFTHE DRAWINGS

Further advantages and aspects of the invention are described in the claims and in the following description of preferred examples of the invention, which are explained in the following by means of the figures.
Figs. 1 shows a first embodiment of an exoscope system according to the invention having a regular screen as viewing device.
Figs. 2 and 3A to 3C show a first mode of operation of the exoscope system.
Figs. 4 and 5A to 5B show a second mode of operation of the exoscope system.
Fig. 6 shows another embodiment of the invention with a head mounted display as viewing device.
Fig. 7 shows a further embodiment of the invention with a regular screen as viewing device and with a head mounted AR device or at least a head mounted eye tracker.
Fig. 8 shows an embodiment of the invention with an ocular system as viewing device.
Fig. 9 shows an embodiment of the invention with a regular screen as viewing device as well as with a shutter goggle to enable stereoscopic viewing.
Fig. 10 to 13 show how the exoscope system is being controlled.
Fig. 14A and 14B show a first embodiment of a camera arrangement with three camera units for the exoscope systems described before.
Fig. 15 shows a second embodiment of a camera arrangement with five camera units for the exoscope systems described before.
Fig. 16 shows a third embodiment of a camera arrangement for the exoscope systems described before having twenty five camera units.

### DETAILED DESCRIPTION OF THE EXAMPLES

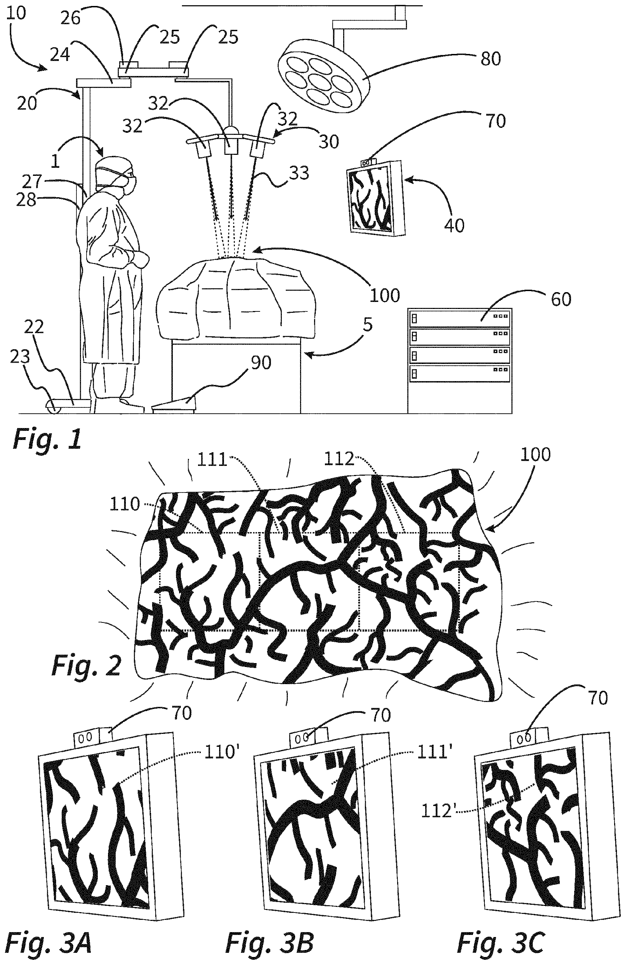

Fig. 1 shows an operating room with an operating table 5 which is additionally equipped with an exoscope system 10 according to the invention.

The main component of this exoscope system 10 is a camera arrangement 30 being positioned above the operating table 5. The camera arrangement 30 faces downwardly to the operating field 100 which is the area of the patient where the operation takes place. Usually the distance between the camera arrangement 30 and the operating field 100 should be between 20 cm and 70 cm.

The camera arrangement 30 is being mounted on a support system 20. Said support system 20 according to this first embodiment is a floor based system. It therefore comprises a bottom base 22 which in the embodiment of fig. 1 is movable due to rollers 23. These rollers can be blocked to inhibit further movement. On the bottom base 22 a support arm 24 is mounted at the end of which said camera arrangement 30 is mounted.

In order to move the camera arrangement 30 to an appropriate position for the operation, multiple elements of the support arm can be adjusted. The support arm 24 has a telescopic section 27 with which the camera arrangement 30 can be moved up and down. Furthermore the support arm 24 has a plurality of joints 25 at which segments of the support arm 24 can be tilted. In the embodiment of fig. 1 electric motor units 26, 28 are provided in order to allow manual movement of the arm with assistance of said motors. Simple embodiments can also be provided without electric motors but rather be intended for strictly manual movement.

Furthermore the camera arrangement 30 may be connected to the support system 20 rotatable about the vertical axis.

Usually the movability of the camera arrangement 30 as a whole is being used only once before the beginning of the operation and afterwards remains on its location, but depending on the type of operation and the size of the operating field repositioning of the camera arrangement can also be needed during operation and can be achieved manually or electrically.

Besides the camera arrangement 30 and the support system 20 for supporting and positioning said camera arrangement 30 the further main components of the exoscope system 10 are a viewing device 40, a controller 60 and a detection system 70 for detecting eye movement, viewing direction and/or head movement of the surgeon 1.

The controller 60 can comprise a regular personal computer connected to the detection system 70 via USB and to the viewing device 40 via HDMI, DVI, Display-Port or other typical video standards.

In the embodiment of fig. 1 the viewing device 40 is a regular screen which for example may have a diagonal of 40 inches (102 cm). Above said viewing device a sensor is being provided which serves as main part of the detection system 70 for detecting eye movement, viewing direction and/or head movement of the surgeon. It can therefore be provided with at least one camera. Furthermore an infrared projector can be part of the detection systems 70 for projecting a pattern which can easily be captured by said camera and easily processed by the controller 60. The controller 60 evaluates the captured image data of said camera of the detection system 70 in order to detect the head position and/orthe recognition of the viewing direction of the surgeon 1.

As mentioned above the camera arrangement 30 is the main component of the exoscope system 10. Said camera arrangement 30 of fig. 1 comprises three distinct camera units 32 wherein each camera unit 32 can be a stereo camera unit 32 with two individual cameras, one for the surgeon's left eye and one for the surgeon's right eye. The camera arrangement is shown in greater detail in later figures.

The three camera units 32 are all having a specific image region 110, 111, 112 adjacent to each other or slightly overlapping. To achieve this the three camera units are aligned such that their respective viewing directions 33 converge.

In fig. 2 the operating field 100 is being shown as well as the three image regions 110, 111, 112. As it can be seen from this figures these image regions 110, 111, 112 do not overlap but abut flush against each other. However, an overlapping configuration is also possible and usually preferred.

As it is shown in fig. 3A to 3C the different image regions 110, 111, 112 captured with the three camera units 32 can be displayed on the viewing device 40 leading to displayed pictures 110', 111', 112'. By having an overlapping configuration of the camera units 32 as mentioned above , there is no risk that a point of interest is right in an intermediate region between two image regions 110, 111, 112.

By moving his head or his viewing direction the surgeon can change between the displayed pictures 110', 111', 112'.

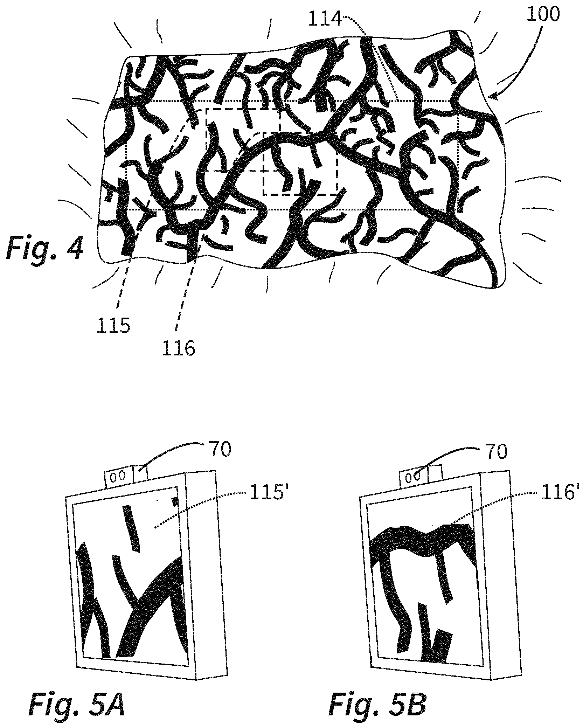

In fig. 4 the operating field 100 is being shown like in fig. 2. The three image regions 110, 111, 112 are the same, wherein in fig. 4 a common image region 114 of all three camera units 32 comprising all three image regions is shown. A corresponding common picture of the common image region is generated internally by the controller 60 by image stitching.

As it is shown in fig. 4, 5A and 5B a partial segment on a variable position 115, 116 of the picture of the common image region 114 can be displayed on the viewing device 40 leading to displayed pictures 115', 116'.

By moving his head or his viewing direction the surgeon can move his gaze over the common picture by moving the partial segment from one position 115 to another position 116 when the area of interest changes.

For detection of such movement the detection system 70 and the controller 60 for evaluation the captured data from the detection system 70 are provided. Although it is preferred that primarily the eye movement and the viewing direction are evaluated it is also possible to use the head movement as main control aspect. Furthermore both, the eye movement and the head movement, can be combined in order to control what is shown on the viewing device.

In a first mode of operation the controller 60 can execute a procedure in which a head or eye movement to the right or to the left is interpreted as command to change the active camera unit 32 so that the next camera unit 32 on the left or on the right respectively is made the active camera unit 32 and the image on the viewing device is replaced by the image of the corresponding image region 110, 111, 112. In a second mode of operation the controller 60 can execute a procedure in which a head or eye movement to the right, to the left, to the top or to the bottom is interpreted as command to change the position 115,116 of the partial segment shown on the viewing device is repositioned accordingly.

In order to avoid accidental commands by an unconscious head movement a further controller may be provided. In case of the embodiment of fig. 1 a pedal 90 is provided for this purpose. This pedal 90 is also connected to the controller 60 and is integrated such that a change of the image region 110, 111, 112 shown on the viewing device 40 is only possible when the pedal is pressed. Therefore in order to change the displayed image region the surgeon presses the pedal and afterwards moves the head to make the controller 60 switch the active camera unit 32 and display the image region of said new active camera unit 32 or to show another segment of a common picture comprising all three pictures 110', 111', 112' together. Another possibility to avoid accidental commands can be provided by setting threshold values, for example such that slight movements are not interpreted as change or movement command regarding the shown region or segment and that only severe movements or specific movement combinations are regarded as movement commands. Depending on the specific needs the behavior could also be contrary thus not changing the view on large head movements as such large movement occur when the surgeon 1 turns to other participants. In this case only small movement are regarded as commands.

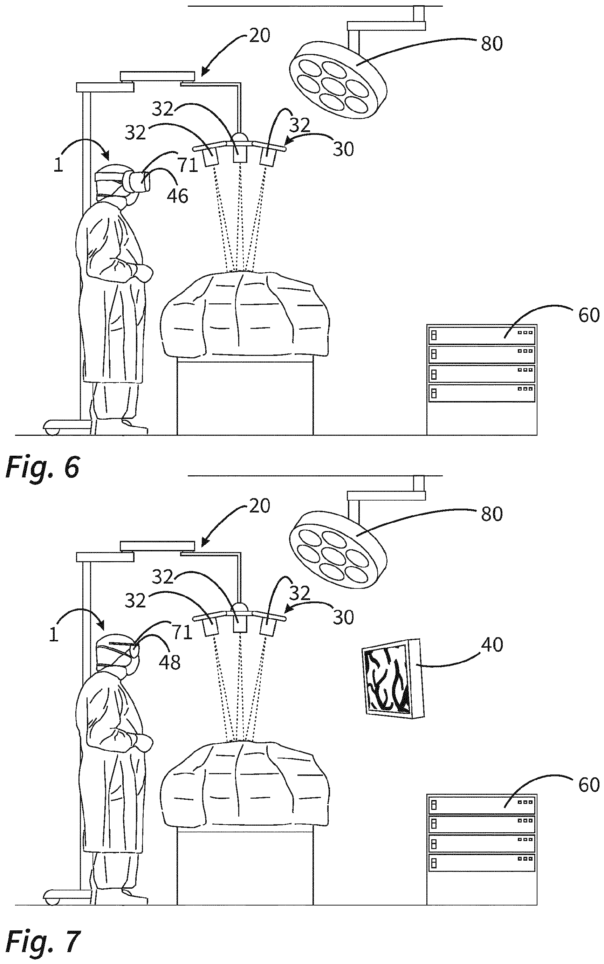

In the embodiment of fig. 6 the viewing device is a head mounted display (HMD) 46 being worn by the surgeon 1. This head mounted display 46 also includes an eye-tracking system 71 that allows the controller 60 to detect the surgeon's 1 viewing direction. On basis of this viewing direction the controller 60 switches between the camera units 32 and the corresponding image regions 110, 111, 112 or moves the segment shown on the HMD display within the common stitched picture.

In the embodiment of fig. 7 the exoscope system 10 also comprises a head mounted unit, but in this case it is an AR unit 48 with an eye tracker 71. Thus the surgeon 1 can see the regular screen 40 as well as overlaying information and/or control elements displayed in the AR unit. The eye tracker 71 included in the AR unit works as the eye tracker 71 in the head mounted display (HMD) according to the embodiment of fig. 4.

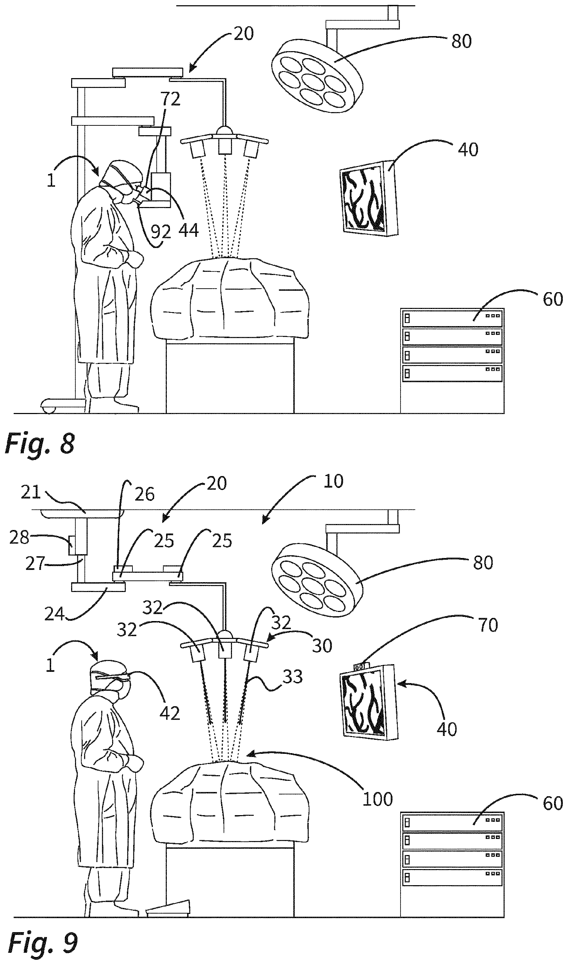

In the embodiment of fig. 8 the viewing device is an ocular system 44 with two oculars. This ocular system is also mounted to the support system 20. It is provided with an eye tracking system 72. The screen 40 is provided as additional viewing device primarily for other participants but can also be used by the surgeon 1 instead of the ocular system 44.

Integrated in the same subunit together with the ocular system 44 is a mouth control system 92 consisting of a lever the surgeon is able to move using his mouth and tongue. Just like the pedal 90 in the previous embodiments this mouth control system 92 enables the surgeon 1 to activate additional functions, especially an enablement/disablement regarding for the head control or eye control function for selecting the picture displaying in the ocular system 44.

In fig. 9 a further embodiment of an exoscope system 10 according to the invention is shown. The main difference to the previous embodiments is, that in this case the support system 20 is mounted to the ceiling and therefore has a ceiling side base 21 mounted in fixed manner to that ceiling. This support system 20 also comprises a support arm 24 with joints 25 and a telescopic section 27 for flexible movement of the camera arrangement 30 relatively to the operating field 100.

The viewing device in this embodiment of fig. 9 is again a regular screen 40 on which an eye tracker and/or head tracker 70 is mounted. In this case the screen is a screen capable to show 3D content by continuously alternating between the picture for the right eye and the picture for the left eye. The surgeon 1 as well as other participants of the surgical operation wear shutter goggles which can hide the view for the right and the left eye and which switch between the eyes synchronized with the screen 40 so that the image is perceived as 3D image.

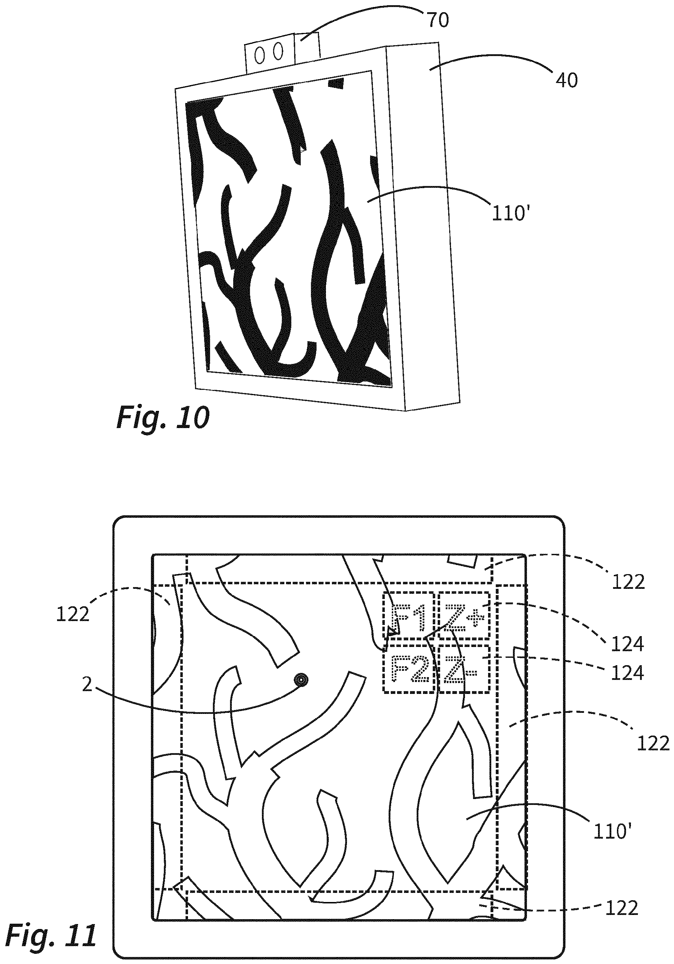

The fig. 10 to 13 illustrate the control on basis of tracked eye or head movement. Fig. 8 shows again a viewing device which in this case is a regular screen 40 with an eye tracking unit 70 mounted on top but could also be a screen of a head mounted display (HMD) or an ocular system as described above.

On the screen the captured picture of one of the camera units 32 is being shown as it can also be seen in fig. 11. Furthermore an overlay layer is being shown, comprising a pointer 2 showing the viewing direction of the surgeon 1 captured by the eye tracker 70, 71, 72 described before, as well as control elements 122, 124. While the control elements 124 are buttons being shown on said overlay layer in order to be focused by the surgeon 1 the control elements 122 are special zones for switching the active camera unit or for moving the shown segment of a common picture of all camera units 32. These switching or movement zones 122 do not necessarily have to be shown on the overlay layer.

If the surgeon 1 turns his gaze on one of the switching or movement zones 122 and keeps his gaze there for a predetermined time, for example for 3 seconds, the controller 60 regards this as switching command and will thus in first mode of operation makes the adjacent next camera unit 32 to the currently active camera unit to the new active camera unit 32 and therefore switches the image to the next image region. In the second mode of operation described above the surgeon's gaze on the switching or movement zones 122 instead moves the partial segment of the common picture according to the zone 122 the surgeon looks at.

Therefore the surgeon is able to move his gaze over the whole operating field just by jumping between the camera units 32 using the switching zones 122 or by scrolling over the common picture using the movement zones 122. The exoscope system 10 could also be designed in such a way that the switching between the camera units 32 or the scrolling does not take place after a predetermined time, but when the pedal 90 is pressed or the mouth controlled device 92 is triggered while the surgeon looks at the respective switching or movement zone 122.

The control elements 124 can be used for additional functions like zoom functionality or other functions like changing the light level of the operating lamp 80. The light level of the this operating lamp 80 shown in the embodiments 1 and 6 to 9 or another light source being held by the support system 20 can also be adjusted automatically by the controller 60 depending on the region the surgeon 1 looks at and thus depending on where the pointer 2 is positioned.

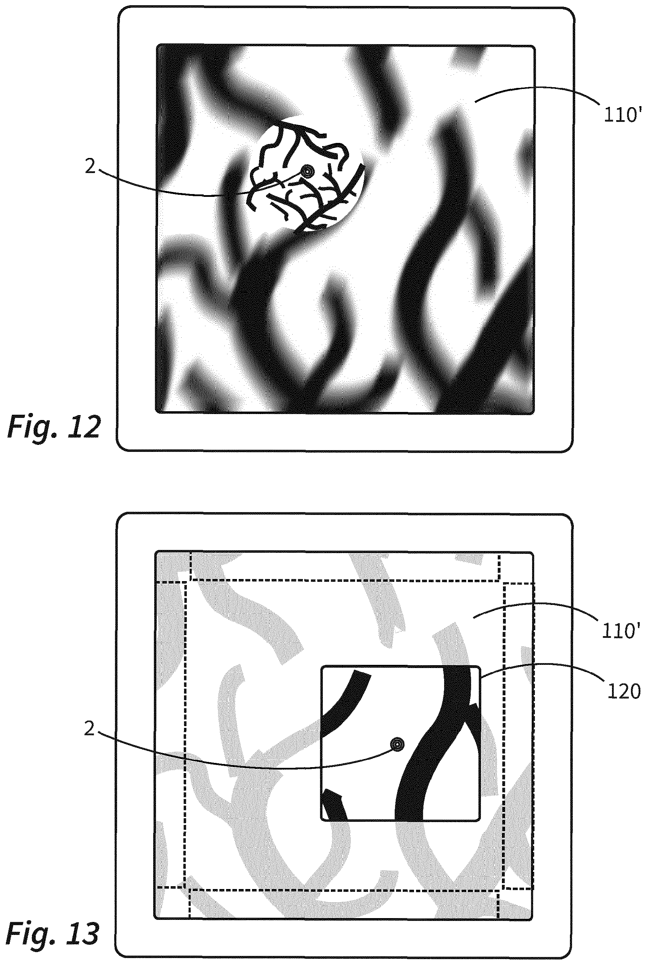

Further functionality which might be controlled by using the control elements 124 can be the focus of the camera units 32. This includes the possibility to activate or deactivate auto focus. The result of this auto focus automatically focusing on the gazed region is shown in fig. 12.

Furthermore it is also possible to mark or highlight regions. This is shown in fig. 13

The controller 60 can furthermore provide a profile system capable of storing presets regarding various values, for example the light level. Furthermore such presets can in particular refer to specific locations of the operating field 100 and corresponding appropriate zoom levels. The surgeon 1 can add presets during operation and then easily return to the specific locations as on demand the controller changes to the respective active camera unit 32 or scrolls the partial segment shown on the viewing device to the stored positon.

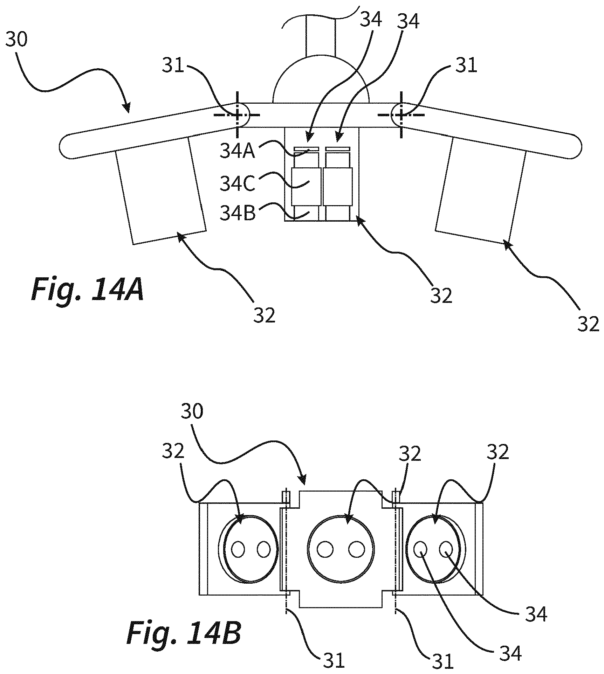

In fig. 14A and 14B the camera arrangement 30 is shown with greater detail. This specific camera arrangement 30 is a rather simple camera arrangement having only three camera units 32. Each of those camera units 32 is capable of capturing stereoscopic images by using two separate cameras 34, one for the left eye's view, one for the right eye's view. Each separate camera has a CCD or CMOS image sensor 34A as well as a lens system 34B and an electric focusing unit 34C.

The camera units 32 are hinged to each other and are thus titlable to each other about a corresponding axis 31. Thus also the viewing direction of the camera units 32 can be adjusted in order to get an appropriate setting for the operating field 100 depending on the size of the operating field 100 and the distance between the operating field and the camera arrangement.

The camera arrangement 30 can be adjusted manually but it is also possible to assign an electric motor to each of the axes 31 in order to fully automatically change the relative position of the camera units 32 before or during an operation. Furthermore also an assisted mode is possible in which these electric motors cause a relative movement of the camera units 32 in reaction of a force manually applied to the camera units 32.

One case in which an electric controller 60 controlling tilting movement is helpful, is in case of using a zoom functionality. As the camera units 32 should cover a continuous region of the operating field and as the image region of the camera units 32 decrease when zooming in using optical zoom, the automatic electric tilting can preserve the flush or slightly overlapping relative position of the image regions 110, 111, 112.

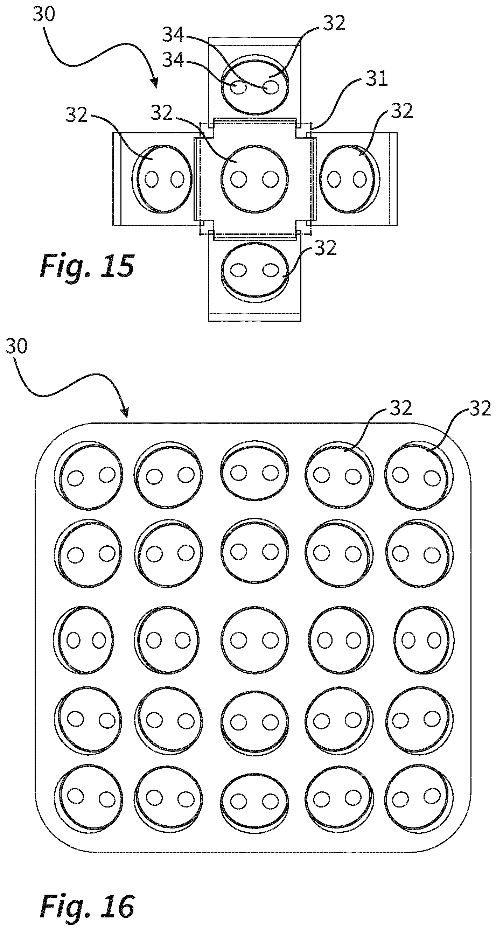

In fig. 15 a further camera arrangement 30 is shown having five camera units 32 in a cross arrangement. Four of these camera units 32 are hinged to a central camera unit 32 and can therefore be adjusted regarding their relative angle.

Fig. 16 shows a further example of a camera arrangement 30. In this example in total 25 camera arrangements are provided being arranged in a 5 x 5 matrix. In this embodiment the camera units 32 are not movable relatively to each other but only the camera arrangement as a whole is movable using the support system 20.

## Claims

1. Exoscope system (10) with a camera arrangement (30) and a viewing device (40, 42, 44, 46, 48) with the following characteristics:
a. the exoscope system (10) has a camera arrangement (30) for capturing the image of an operating field (100), and
b. the exoscope system (10) has a support system (20) for positioning the camera arrangement (30) above the operating field (100), and
c. the exoscope system (10) has a viewing device (40, 42, 44, 46, 48) for displaying an image region (110, 111, 112) of the operating field (100) which is captured by the camera arrangement (30),
**characterized by** the following additional features:
d. the camera arrangement (30) comprises at least two camera units (32) which are directed or can be directed to capture pictures (110', 111', 112') of at least two different image regions (110, 111, 112) of the operating field (100), and
f. the controller (60) comprises a detection system (70, 71, 72) for detecting eye movement, viewing direction (2) and/or head movement of an operator (1), and
e. the exoscope system (10) includes a controller (60) for selecting an image region (110, 111, 112) from said at least two image regions (110, 111, 112) and for displaying its picture on the viewing device (40), wherein the controller (60) is designed to switch between the at least two image regions (110, 111, 112) on the viewing device (40) as a function of the detected eye movement, viewing direction (2) and/or head movement,
and/or
the exoscope system (10) includes a controller (60) for generating a common picture (114) by stitching together the pictures (110', 111', 112') of the image regions (110, 111, 112) and for displaying a partial segment (115, 116) of this common picture on the viewing device (40), wherein the controller (60) is designed to change the partial segment (115, 116) of the common picture shown on viewing device (40) as a function of the detected eye movement, viewing direction (2) and/or head movement.

2. Exoscope system (10) according to claim 1 with the following additional characteristic:
a. the camera units (32) are each designed as stereo camera units (32) with two individual cameras (34),
preferably with at least one of the following additional characteristics:
b. each individual camera (34) has its own image sensor (34A), which in particular is preferably a CCD image sensor or CMOS image sensor, and/or
c. each individual camera has a lens system (34B); and/or
d. each individual camera has a motor-driven focus unit (34C).

3. Exoscope system (10) according to claim 1 or 2 with the following additional characteristic:
a. the camera arrangement (30) comprises at least three camera units (32).

4. Exoscope system (10) according to one of the above claims with the following additional feature:
a. the at least two camera units (32) of the camera arrangement (30) are movable relative to each other and can be fixed in a plurality of relative arrangements,
preferably with at least one of the following additional characteristics:
b. the camera units (32) are movable relative to each other with motor support, and/or
c. the camera units (32) are purely manually movable relative to each other.

5. Exoscope system (10) according to one of the above claims with the following additional feature:
a. the camera units (32) have a converging viewing direction (33) or are adjustable in a relative arrangement in which they have a converging viewing direction (33).

6. Exoscope system (10) according to one of the above claims with the following additional features:
a. the exoscope system (10) comprises at least one light source (80) for illuminating the operating field,
b. the controller (60) is designed to adjust the brightness of the at least one light source (80) as a function of the detected eye movement, viewing direction (2) and/or head movement.

7. Exoscope system (10) according to one of the above claims with the following additional characteristic:
a. the controller (60) is adapted to adjust the focus of the camera units (32) as a function of the detected eye movement, viewing direction (2) and/or head movement.

8. Exoscope system (10) according to one of the above claims with the following additional feature:
a. the controller (60) is designed to display a marking or highlighting (120) in the displayed image region (110') as a function of the detected eye movement, viewing direction (2) and/or head movement.

9. Exoscope system (10) according to one of the above claims with the following additional feature:
a. the controller (60) is designed to display control elements (122, 124) on the viewing device (40, 42, 44, 46, 48) and to select control elements as a function of the detected eye movement, viewing direction (2) and/or head movement,
preferably with at least one of the following additional characteristics:
b. at least one control elements (124) is provided for selecting a zoom level and/or
c. at least one control elements (124) is provided for setting a motor-driven focus unit (34C).

10. Exoscope system (10) according to one of the foregoing claims with the following additional characteristics:
a. the support system (20) is a support system (20) for installation on a floor or on a wall, and
b. the support system (20) has a bottom base (22) or wall base, and
c. the support system (20) has a support arm (24) extending upwardly from the bottom base (22) and/or extending from the side over the operating field (100), at the end of which the camera arrangement (30) is mounted,
preferably with at least one of the following additional characteristics:
d. a plurality of rollers (23) is provided on the bottom base (22), the rollers (23) preferably being at least partially blockable by means of a blocking device, and/or
e. the support arm (24) has at least one joint (25) which permits adaptability of the positioning and/or alignment of the camera arrangement (30), at least one joint (25) preferably being provided with a motor drive (26), and/or
f. the support arm (24) has at least one telescopic section (27) which is variable in length and permits adaptability of the positioning and/or orientation of the camera arrangement (30), preferably the telescopic section (27) being provided with a motor drive (28).

11. Exoscope system (10) according to any of claims 1 to 9 with the following additional characteristics:
a. the support system (20) is a support system (20) for mounting on the ceiling, and
b. the support system (20) has a base (21) on the ceiling side, and
c. the support system (20) comprises a support arm (24) extending downwardly from the base (21) and extending over the operating field (100) and at the end of which the camera arrangement (30) is attached,
preferably with at least one of the following additional characteristics:
d. the support arm (24) has at least one joint (25) which permits adaptability of the positioning and/or alignment of the camera arrangement (30), at least one joint (25) preferably being provided with a motor drive (26), and/or
e. the support arm (24) has at least one telescopic section (27) which is variable in length and permits adaptability of the positioning and/or orientation of the camera arrangement (30), preferably the telescopic section (27) being provided with a motor drive (28).

12. Exoscope system (10) according to one of the previous claims with the following additional characteristic:
a. the viewing device (40) comprises a screen (40),
preferably with the following additional characteristic:
b. the exoscope system (10) comprises at least one shutter goggle (42).

13. Exoscope system (10) according to one of the above claims with the following additional feature:
a. the viewing device (40) comprises at least one ocular (44),
preferably with at least one of the following additional characteristics:
b. the viewing device (40) comprises two oculars (44) and/or
c. an eye-tracking system (72) for detecting the viewing direction (2) is integrated into the at least one ocular (44).

14. Exoscope system (10) according to one of the above claims with the following additional feature:
a. the viewing device (40) comprises a head-mounted unit (HMD) (46) with integrated screen.

15. Exoscope system (10) according to one of the above claims with the following additional feature:
a. the exoscope system (10) comprises at least one mouth or foot controlled input device (90) as part of the controller (60).

16. Use of an exoscope system (10) according to one of the above claims for visual enlargement of the surgical field (100) in a neurosurgical operation, in an ophthalmic operation, in an ENT operation, in a plastic or reconstructive operation or in a dental operation.
